# EUROPEAN PATENT SPECIFICATION

(11) **EP 1 796 628 B1**
(45) Date of publication and mention of the grant of the patent: **23.07.2008**
(21) Application number: 05801857.3
(22) Date of filing: 28.09.2005
(51) Int. Cl.: A61K 9/06, A61L 31/04, A61P 27/02, A61P 27/04

(54) **NEW VISCOELASTIC COMPOSITION COMPRISING ALGINATE AND VITAMIN E TPGS OR TPGSA**
NEUE VISKOELASTISCHE ZUSAMMENSETZUNG UMFASSEND ALGINAT UND VITAMIN E TPGS ODER TPGSA
NOUVELLE COMPOSITION VISCOELASTIQUE COMPRENANT UN ALGINATE ET UNE VITAMINE E TPGS OU TPGSA

(30) Priority: 29.09.2004 US 614274 P
(43) Date of publication of application: 20.06.2007
(73) Proprietor: BAUSCH & LOMB INCORPORATED, Rochester, NY 14604-2701 (US)
(72) Inventor: XIA, Erning, Penfield, NY 14526 (US); GREEN, George, Victor, NY 14564 (US)
(74) Representative: Vossius & Partner
(86) International application number: PCT/US2005/035133
(87) International publication number: WO 2006/039458

(56) References cited:
- WO-A-00/35432
- WO-A-03/057187
- WO-A-20/05046641
- WO-A-20/05082333
- WO-A-20/05097225
- US-A- 5 366 964
- US-A- 5 603 929
- US-A- 5 792 103
- US-A- 5 880 107
- US-A- 5 886 030
- US-A1- 2003 068 250
- HYNDIUK R A ET AL: "Overview of the corneal toxicity of surgical solutions and drugs: and clinical concepts in corneal edema." LENS AND EYE TOXICITY RESEARCH. 1992, vol. 9, no. 3-4, 1992, pages 331-350, XP009060023 ISSN: 1042-6922
- SCHWENN O ET AL: "Healon5 versus Viscoat during cataract surgery: intraocular pressure, laser flare and corneal changes." GRAEFE'S ARCHIVE FOR CLINICAL AND EXPERIMENTAL OPHTHALMOLOGY = ALBRECHT VON GRAEFES ARCHIV FUR KLINISCHE UND EXPERIMENTELLE OPHTHALMOLOGIE. OCT 2000, vol. 238, no. 10, October 2000 (2000-10), pages 861-867, XP002363182 ISSN: 0721-832X
- SILVER F H ET AL: "PHYSICAL PROPERTIES OF HYALURONIC ACID AND HYDROXYPROPYLMETHYLCELLUL OSE IN SOLUTION; EVALUATION OF COATING ABILITY" JOURNAL OF APPLIED BIOMATERIALS, JOHN WILEY & SONS, INC., NEW YORK, NY, US, vol. 5, no. 1, 1994, pages 89-98, XP008050195 ISSN: 1045-4861

## Description

This application claims the benefit of Provisional Patent Application No. 60/614,274 filed September 29, 2004 and is incorporated herein by reference.

This invention relates to a viscoelastic composition, and related device used in viscosurgical applications and more particularly to a viscoelastic composition used in ophthalmic surgical application such as cataract removal surgery. The use of the viscoelastic composition is also described.

In the past decade, advances in the technology of eye surgery have made surgical treatment of eye disease and deformities attractive to alternative therapies. Cataract removal is one of the more common surgical procedures. Cataracts are opacities of the ocular lens, which generally arise in the elderly. Typically, cataract surgery involves removal of the cataractous lens from the capsular bag and replacement of the cataractous lens with a synthetic intraocular lens. Presently, this procedure involves making an incision through the sclera into the anterior chamber of the patient's eye. Another incision is made into the capsular bag. The cataractous lens is fractured in the capsular bag by procedures such as phacoemulsification and removed from the capsular bag by procedures such as aspiration. Thereafter an intraocular lens is inserted into the capsular bag and deployed therein.

The overall procedure is potentially traumatic to the capsular bag and the tissue surrounding the anterior chamber. It is advantageous to reduce the amount of trauma to any living tissue in the patient's eye during a surgical procedure. Particularly, corneal endothelial cells are sensitive to damage. Damage to the corneal endothelial cells is permanent. Serious damage can eventually lead to corneal tissue damage and loss of eyesight.

Moreover, the process of phacoemulsification produces free radicals and/or oxidants. Free radicals and/or oxidants are unstable and react somewhat indiscriminately with biological molecules in tissue. For example, free radicals and/or oxidants that are produced in phacoemulsification can damage proteins, cell walls or even the DNA of a cell. It is advantageous to reduce the damage caused by these free radicals and/or highly reactive ions.

Viscoelastic compositions are injected in the anterior chamber of the eye and the capsular bag during surgery to protect the tissue from physical trauma. The viscoelastic compositions provide a physical barrier or cushion between the instruments and the tissue. Furthermore, viscoelastic compositions maintain the shape of a cavity during operation including the anterior chamber and capsular bag. Viscoelastic compositions have been known to contain agents that are free radical scavengers and/or antioxidants.

Selection of an ingredient in a viscoelastic composition for the purpose of controlling free-radical activity and/or antioxidants requires satisfying several criteria. The ingredient cannot negatively impact the viscoelastic properties, irritate tissue or cause an adverse immune response. The ingredient should be effective as a free-radical scavenger and/or antioxidant under conditions of desirable pH and osmolality. Of course, the effectiveness of the free-radical scavenger to dampen free radical activity is an important factor.

WO 2000/35432 describes a viscoelastic composition comprising an amount of one or more viscoelastic agent(s), an amount of one or more physiological antioxidant(s) and an amount of one or more compound(s) having a specific formula (I). The viscoelastic compositions are stated to be useful in the prevention or treatment of inflammatory and proliferative events incident to ocular surgery.

U.S Patent No. 5,880,107 discloses a viscoelastic composition for use in eye surgery. The viscoelastic composition contains hyaluronic acid as the primary ingredient to provide appropriate viscoelasticity. The composition further contained a citric acid salt, typically tri-sodium citrate, an antioxidant tolerated by the intraocular tissues and a phosphate buffer. The antioxidant was selected from the group comprising glucose, sulphides, superoxide dismutase (SOD), cysteine and derivates thereof. Furthermore, other antioxidants that could be used include antioxidants, which have at least one -SH or -CHO group, peptides and enzymes.

US Patent No. 6,086597 discloses a sodium hyaluronate viscosurgical composition that contains a compound as a scavenger including superoxidedismutase, mannitol and glutathione. Furthermore, the use of Vitamin E (tocopherol) as an antioxidant in a viscosurgical composition is known in the art.

U.S. Pat. Nos. 5,603,929 and 5,653,972 disclose preserved, storage-stable ophthalmic, compositions comprising acidic drugs, a polymeric quaternary ammonium compound and boric acid, and methods for controlling ocular inflammation using such compositions. Vitamin E tocopherol polyethylene glycol 1000 succinate is disclosed as a formulation component. U.S. Pat. No. 5,886,030 discloses the use of Vitamin E tocopherol derivatives, including Vitamin E tocopherol polyethylene glycol 1000 succinate, in anti-inflammatory ophthalmic compositions. Methods are disclosed for treating or controlling ocular inflammation and for improving comfort and reducing irritation in compositions containing ophthalmic therapeutic agents, which are irritating to the eye. The compositions may include preservatives such as benzalkonium chloride, Polyquad® and Dymed® (polyhexamethylenebiguanide).

U.S. Patent Application No. 2003-0068250 discloses the use of a vitamin derivative component, including vitamin E tocopherol polyethylene glycol succinate (TPGS), with a preservative in an ophthalmic preservative composition.

While significant improvements have been made in the rheological properties of viscoelastic compositions, there still exists a need for a composition that reduces the free radical and/or oxidant activity without negatively impacting the viscoelastic properties of the viscoelastic composition. The present invention addresses these and other needs.

The present invention relates to a viscoelastic composition that comprises an aqueous solution of an alginate viscoelastic polymer and a soluble vitamin derivative, wherein the soluble vitamin derivative includes Vitamin E TPGS or Vitamin E TPGS A. The composition is an effective viscosurgical device for use in cataract surgery to maintain the shape of the anterior chamber or the capsular bag. The viscoelastic polymer is present in an amount from a minimum of about 0.01 %w/v to a maximum of about 20%w/v based upon the total volume of the viscoelastic composition.

The present invention also describes a method of temporarily maintaining space in a cavity in human tissue. The method comprises the step of injecting the viscoelastic composition of one or more embodiments of the present invention into the cavity. The viscoelastic composition, then, is removed from the cavity. Preferably, the cavity is the anterior chamber of the eye or the capsular bag.

In another embodiment, a method of protecting tissue from trauma during a surgical procedure is disclosed. The method includes coating at least a portion of the tissue with a viscoelastic composition of any one embodiment of the present invention. A surgical procedure is performed near the tissue after the coating. After the surgical procedure, at least a portion of the viscoelastic composition is removed from the tissue.

In still another embodiment, a method of replacing a natural lens from an eye is described. The method comprises providing a passage through a sclera into an anterior chamber of the eye. At least a portion of the aqueous humor is removed from the anterior chamber. A viscoelastic composition according to one or more embodiments of the present invention is inserted into the anterior chamber. The corneal lens is phacoemulsified in the capsular bag. The entire lens is removed substantially from the capsular bag. The viscoelastic composition is inserted into the capsular bag. An intraocular lens is then inserted into the capsular bag.

In another embodiment, a package for a viscoelastic composition is disclosed, the package comprising a syringe containing a viscoelastic composition according to any embodiment, aspect, feature, combination or concept disclosed herein.

### Introduction

The present invention is directed to a viscoelastic composition comprising an aqueous solution having from a minimum of about 0.01 %w/v to a maximum of about 20%w/v of an alginate viscoelastic polymer based upon the total volume of the viscoelastic composition. The viscoelastic composition further contains a soluble vitamin derivative, wherein the soluble vitamin derivative includes vitamin E TPGS or vitamin E TPGSA. "Soluble vitamin derivative" is defined as a vitamin that has been chemically modified by adding a substituent group to the vitamin to form a derivative that has a solubility greater than the original vitamin. The present invention also includes uses and a device. In one embodiment, the solubility of the soluble vitamin derivative is a minimum of about 100µg/ml, about 500µg/ml, about 1000µg/ml or about 5000 µg/ml.

### Definitions

Viscosurgically pure as it pertains to a viscoelastic composition or ingredient thereof is defined as a level of purity that is sufficiently free of impurities to meet or exceed the United States Food and Drug Administration standards for a viscosurgical viscoelastic effective at the time this application is filed.

Polysaccharides are defined as saccharides that have 10 or more saccharide monomer units.

Zero-shear viscosity is defined as the extrapolation of the viscosity of a liquid to a zero-shear rate from measurements of viscosity as the shear rate approaches zero measured on a plate and cone rheometer at 34°C.

High-shear viscosity is defined as the viscosity of a liquid measured on a plate and cone rheometer at 34 °C with a shear rate of 300 S⁻¹.

Pseudoplastic material is defined as a material that has relatively high viscosity under low-shear and relatively low viscosity under high-shear conditions.

The phrase, "removing substantially all" as it relates to lenses and lens fragments is defined as removing a sufficient quantity that an effective implantation of an intraocular lens is not inhibited thereafter. According to one embodiment, an effective removal of the lens requires a minimum of 90%w/v of the lens, 95%w/v of the lens or 98%w/v of the lens.

A cannula is defined as any tubular member having a passage that is configured to penetrate tissue and deliver a device through the passage.

The percentage of quenching as describe in the application with the exception of the examples is defined as the percentage amount that free-radical activity is prevented as evaluated by the 2-deoxy-D-ribose (2-DR) oxidation method. This is a conventional method of OH-radical detection forming by the Fenton reaction, radiation or ultrasound. It is based on its reaction with 2-DR, the obtained product of degradation, after a thermoactivated reaction with thiobarbituric acid (TBA) produces a pink chromogen quantified by HPLC.

### Formulation

According to one embodiment of the present invention, a viscoelastic composition comprising an aqueous solution having from a minimum of about 0.01%w/v to a maximum of about 20%w/v of an alginate viscoelastic polymer based upon the total volume of the viscoelastic composition is described. The viscoelastic composition further contains soluble vitamin derivative, wherein the soluble vitamin derivative includes vitamin E TPGS or vitamin E TPGS A.

As used herein, a vitamin derivative component or vitamin-based surfactant component includes a vitamin derivative with a chemical structure such that the resulting moiety is effective as a surfactant in the present compositions and/or uses. Without wishing to limit the invention to any particular theory of operation, it is believed that the vitamin derivative components of the present invention form micelles and have a critical micelle concentration. Such components can at least assist in cleaning contact lenses in or out of the eye, can at least assist in removing, preferably can remove foreign particulates, debris, antigens and the like from the eye and are ophthalmically acceptable and/or substantially non-toxic to the eye.

In addition to the Vitamin E derivatives mentioned above, Vitamins A, A2, C, D1, D2, D3, D4, E, K1, K2 and folic acid are preferred vitamins, which can be derivatized to form surfactants. Any suitable vitamin derivative can be employed provided such derivative is effective to function as described herein, for example, as a surfactant. Examples of such derivatives include, but not limited to, the succinic acid ester or amide of Vitamin A (retinol), which can be prepared and then further esterified or amidated (e.g., coupled with an amide bond) with a polyalkylene glycol, for example, selected from polyethylene glycols, polypropylene glycols, mixtures thereof and the like, to form a suitable surfactant. Polyethylene glycol 1000 is one very useful polyalkylene glycol. Vitamins D1, D2, D3 or D4 can be similarly esterified or amidated to form suitable surfactants. The dihydro derivatives of Vitamins K1 and K2 can be prepared and thereafter esterified or amidated with succinic acid and polyalkylene glycol, e.g., polyethylene glycol 1000. Folic acid can be esterified or amidated at either of its two free carboxylic acid groups by polyalkylene glycol, e.g., polyethylene glycol 1000. Non-anionic Vitamin E derivatives are particularly preferred.

Two very useful Vitamin E derivative-based surfactants are D-α-tocopherol polyethylene glycol 1000 succinate (Vitamin E TPGS), and its amide analogue (Vitamin E TPGSA). The structure of Vitamin E TPGS is represented as follows:

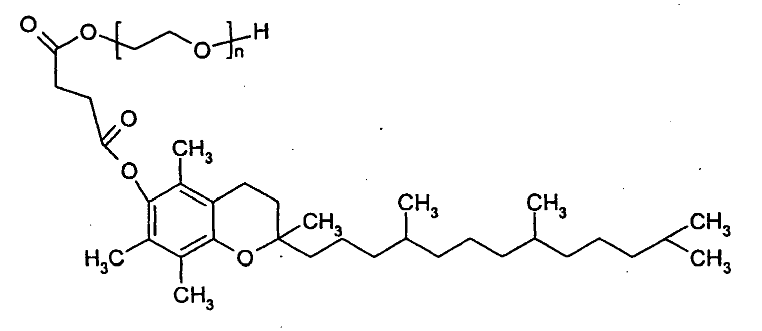

Vitamin E TPGS is a polyethylene glycol (PEG) ester of D-α-tocopherol acid succinate, where the polyethylene glycol (PEG) molecular weight is about 1000. D-α-tocopherol acid succinate is, in turn, a succinic acid ester of D-α-tocopherol, Vitamin E. Vitamin E TPGSA is a polyethylene glycol (PEG) amide of D-α-tocopherol acid succinate containing an amide bond between the PEG chain and the distal succinic acid free acid group, and where the PEG molecular weight is about 1000. The structure of TPGSA is represented as follows.

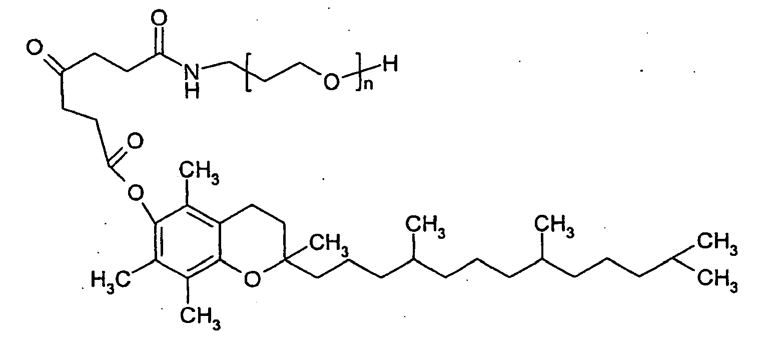

Other Vitamin E-based surfactant components can also be used in the present invention. All of the naturally occurring Vitamin E compounds that exhibit at least part of the biological activity of α-tocopherol and their corresponding synthetic forms can be used to form surfactants for use in the present invention. Vitamin E compounds useful for the present invention include, without limitation, α-, β-, X-, and δ-tocopherol and α-, β-, X-, and δ-tocotrienol. All these compounds occur as a variety of isomers. The commercially available synthetic forms of Vitamin E comprise an approximately equal mixture of eight sterioisomeric forms of α-tocopherol. The presently useful surfactants can be based on a single vitamin isomer or a mixture of vitamin isomers.

A variety of surfactant forms of vitamins such as Vitamin E or other vitamins, can be produced and are useful in the present invention. These surfactants can be produced with known synthetic chemistry methodology, for example, direct esterification or amidation of the phenolic OH group of tocopherol with a surfactant chain such as polyethylene glycol or a second esterification or amidation of a primary-ester intermediate such as Vitamin E succinate. Esterification of the phenolic OH group of tocopherol is a preferred chemical synthetic path. The vitamin-based esters are preferred for use as surfactants in accordance with the present invention. Ester or amide bonds are readily hydrolyzed by enzymes, such as enzymes with esterase or amidose activity in-vivo, which leads directly to the production of biologically active vitamins. Ocular tissues are known to contain esterases and amidoses, which hydrolyze ester and amide bonds, respectively substantially in ocular prodrugs. Thus, ocular tissue esterases or amidoses can act upon the ester or amide bonds in, for example, Vitamin E TPGS and other ester-based vitamin surfactants to release biologically active vitamins to tissues.

Vitamin-based surfactants, which have ester structures, are often sufficiently stable to substantially and effectively maintain surfactant activity in the aqueous compositions, e.g., solutions, of the present invention. Other types of surfactants can be produced with other in-vivo labile bonds, which also lead to the production of biologically active vitamins, provided that such surfactants have sufficient stability in aqueous solution to provide an acceptable level of surfactant activity. Amide bonds, as an example, are estimated to be several orders of magnitude more hydrolytically stable than ester bonds. Nonionic surfactants are preferred. Under certain conditions, for example, where the activity of the disinfecting agent is not compromised and in-eye safety and comfort can be maintained, cationic, amphoteric and anionic surfactants can also be employed. A variety of nonionic surfactant classes may be produced based on a vitamin precursor and used in the present invention, such as the polyethylene glycol surfactants. Other nonionic surfactant classes corresponding to the presently useful vitamin- based surfactant components include, without limitation, the polyoxyethylated linear alcohols, nonoxynols, octoxynols, polyoxyethylated dodecylamines, sorbitan monoesters and the like and mixtures thereof.

The surfactants useful in the present invention advantageously are watersoluble when used alone or as a mixture. Such surfactants preferably have HLB values of about 12 to about 13 when used alone. In addition, the surfactant or surfactants employed preferably produce a clear solution.

Vitamin E TPGS is known to be unstable with respect to hydrolysis upon exposure to acidic and alkaline pH conditions. The instability is due to acid- or base-catalyzed hydrolysis of the ester linkages. As pH approaches neutrality in buffered solutions, Vitamin E TPGS becomes more stable. This can be problematic for some compositions, as pH 7.5 is more optimal for ocular comfort, since it is closer to the human tear pH of 7.45. Therefore, compositions of the present invention can also optionally include the hydrolysis products of Vitamin E surfactant ester bond hydrolysis to further stabilize the surfactant ester against hydrolysis during shelf storage. Hydrolysis products of ester forms of other vitamin-based surfactants can also be used in a similar manner to stabilize the surfactant as well as provide an additional source of vitamin.

Hydrolysis of Vitamin E TPGS to Vitamin E tocopherol hemisuccinate and polyethylene glycol can result in a solution, which is incompatible with disinfecting agents such as polyhexamethylene biguanide (PHMB). This is because it has been found that in some cases the amount of Vitamin E tocopherol hemisuccinate anion which forms can substantially ion-pair the cationic PHMB, thus reducing or neutralizing PHMB antimicrobial activity. Hydrolysis of Vitamin E TPGS in aqueous·solution is quite slow and in most cases results in part-per-million concentrations of hydrolysis products. Nonetheless, this may be sufficient to inactivate a disinfecting agent such as PHMB or other cationic antimicrobial agents. In such cases, an alternative amide-based surfactant, such as Vitamin E TPGSA, may be advantageously employed. It may be useful to suppress hydrolysis of the Vitamin E TPGS. Such suppression may be achieved by employing small amounts of polyethylene glycol, for example, PEG 1000, and succinic acid, buffering the solution at a slightly acidic pH of about 6.8-6.9, and using a sterically-hindered, non-complexing buffer, such as bis tris, 2-(bis(2-hydroxyethyl)amino)-2(hydroxymethyl)-1,3-propanediol. The relatively bulky hydrophilic hydroxyethyl groups of bis tris act to shield the basic nitrogen and make it more difficult for this buffer to serve as a base-catalyst for ester hydrolysis. It is preferred to use more stable amide-based vitamin surfactant in compositions containing conventional amounts of PHMB (for example, about 0.5 ppm to about 1 ppm) where such compositions may otherwise result in unstable Vitamin E TPGS.

The vitamin-based surfactants used in the present invention can also be used together with conventional non-vitamin surfactants, for example, nonionic, cationic, anionic and amphoteric non-vitamin surfactants. The vitamin-based surfactants, if used in combination with one or more non-vitamin surfactants substantially are preferably used with nonionic non-vitamin surfactants.

In one embodiment, the viscoelastic composition has a concentration of soluble vitamin derivative that is a minimum of about 0.01%w/v and/or a minimum of about 4%w/v based upon the total weight of the viscoelastic composition. Typically, the concentration of soluble vitamin derivative is a minimum of about 0.2%w/v and a maximum of about 3%w/v based upon the total volume of the viscoelastic composition. Preferably, the concentration of soluble vitamin derivative is a minimum of about 0.3%w/v, about 0.4%w/v or about 0.5%w/v and a maximum of about 2%w/v, about 1%w/v or about 5%w/v based upon the volume of the viscoelastic composition in one aspect of the invention.

The viscoelastic composition of one embodiment of the present invention has a ratio of the viscosity of the viscoelastic composition to the viscosity of a comparable viscoelastic composition having no soluble vitamin derivative that is from a minimum of about 1 to a maximum of about 2.5. A comparable viscoelastic composition is defined as a viscoelastic composition that has all of the same chemical ingredients as the viscoelastic composition at the same concentrations except it has no soluble vitamin derivative. Typically, the ratio of the viscosity of the viscoelastic composition to the viscosity of a comparable viscoelastic composition is from a minimum of about 1, about 1.1 and about 1.2 to a maximum of about 2.5, about 2.2 and about 2.

The viscoelastic composition of yet another embodiment quenches chemical scavengers effectively, wherein the percentage of quenching is a minimum of about 45%. Typically, the percentage of quenching is greater than about 55%, about 65%, about 70%, about 75% or about 80% according to the method of testing known in the art.

In addition to the alginate viscoelastic polymer, the viscoelastic composition can comprise one or more viscoelastic polymers that are useful and known as viscosurgical devices. In one embodiment, the additional viscoelastic polymer is selected from the group comprising hyaluronic acid, hydroxypropylmethylcellulose, polyacrylic acid, carbopol, polyvinylalcohol, polyvinylpyrrolidone, condroitin sulfate, polycarbophil, methylcellulose, carboxymethylcellulose, hydroxyethylcellulose, hydroxypropylcellufose, ethylcellulose, polyethylene oxides, pectin, xanthan gum, dextrans, collagen and derivatives threof and salts thereof and combinations thereof.

In one embodiment, the average molecular weight of the viscoelastic polymer, including a polysaccharide, is from a minimum of about 20 kD to a maximum of about 5,000 kD. Generally, the average molecular weight of a viscoelastic polymer, including polysaccharide, is from a minimum of about 30 kD, about 50 kD, about 70 kD, about 400 kD, about 500 kD, about 750 kD or about 1,000 kD. Typically, the average molecular weight of a viscoelastic polymer, including a polysaccharide, is up to a maximum of about 50 kD, about 80 kD, about 100 kD, about 200 kD, about 400 kD, about 500 kD, about 1,000 kD or about 3,000 kD.

Typically, there are two general classes of viscoelastic compositions. A dispersive viscoelastic composition has properties that disperse or coat the tissue well and adhere well to the tissue. A dispersive viscoelastic composition (also known as an "adhesive viscoelastic composition") typically has a low molecular weight. A cohesive viscoelastic composition is better at maintaining the space in a cavity in human tissue and is less likely to leak from the cavity under low or zero shear conditions. Typically, a cohesive viscoelastic composition has a high molecular weight.

In one embodiment, the average molecular weight of a viscoelastic polymer in a dispersive viscoelastic composition is from a minimum of about 20 kD, 30 kD, about 50 kD or about 70 kD. Typically, the average molecular weight of a viscoelastic polymer in a dispersive viscoelastic composition is up to a maximum of about 50 kD, about 80 kD, about 100 kD, about 200 kD, about 400 kD or about 500 kD.

In another embodiment, the average molecular weight of a viscoelastic polymer in a cohesive viscoelastic composition is from a minimum of about 400 kD, about 500 kD, about 750 kD or about 1,000 kD. Typically, the average molecular weight of a viscoelastic polymer in a cohesive viscoelastic composition is up to a maximum of about 1,000 kD, 3,000 kD or about 5,000 kD.

The concentration of the viscoelastic polymer is from a minimum amount of about 0.01%w/v and up to a maximum amount of about 20%w/v based upon the total weight of the viscoelastic composition in one embodiment. Typically, the concentration of the viscoelastic polymer is from a minimum of about 0.1%w/v, about 0.2%w/v, about 1.0 or about 2.0%w/v and up to a maximum of about 0.3%w/v, about 0.5%w/v, about 1%w/v, about 2%w/v about 3%w/v, about 5%w/v or about 15%w/v based upon the total weight of the viscoelastic composition.

The viscoelastic polymer comprises alginate. Typically the concentration of alginate is from a minimum of about 0.05%w/v to a maximum of about 9%w/v based upon the volume of the viscoelastic composition. Optionally, the minimum alginate concentration is about 1%w/v, about 1.5%w/v, about 2%w/v, about 3%w/v or about 4%w/v based upon the total weight of the viscoelastic composition. Optionally, the maximum alginate concentration is about 10%w/v, about 8%w/v, about 6%w/v, about 4%w/v, about 3%w/v or about 2%w/v based upon the total weight of the viscoelastic composition. Preferably, the alginate concentration is from a minimum of about 2%w/v to a maximum of about 5.25%w/v.

In one embodiment, the average molecular weight of the alginate is from a minimum of about 50 kD to a maximum of about 5,000 kD. Typically, the average molecular weight of the alginate is from a minimum of about 100 kD, about 200 kD, about 500 kD or about 1000 kD. Typically, the average molecular weight of the alginate is up to a maximum of about 2000 kD, about 1000 kD, about 750 kD or about 500 kD.

The viscoelastic composition has one or more properties including but not limited to osmolality, pH, zero-shear viscosity and high-shear viscosity. The osmolality of the viscoelastic composition is from a minimum of about 200mOsmol/kg to a maximum of about 400mosmol/kg in an embodiment. Typically, the osmolality of the viscoelastic composition is from a minimum of about 220mOsmol/kg, about 260mOsmol/kg, about 280mOsmol/kg, about 300mOsmol/kg or about 320mOsmol/kg to a maximum of about 400mOsmol/kg, about 380mOsmol/kg, about 360mOsmol/kg or about 340mOsmol/kg.

The zero-shear viscosity of the viscoelastic composition is from a minimum of about 6.10⁴ cps to a maximum of about 4.10⁶ cps. Generally, the zero-shear viscosity of the viscoelastic composition is from a minimum of about 6x10⁴ cps, about 4x10⁵ cps or about 8x10⁵ cps to a maximum of about 3.5x10⁶ cps, about 1.8x10⁶ cps or about 1.2x10⁶ cps.

The high-shear viscosity of the viscoelastic composition is from a minimum of about 500 cps to a maximum of about 2000 cps. Generally, the high-shear viscosity of the viscoelastic composition is from a minimum of about 500 cps, about 600 cps or about 700 cps to a maximum of about 2000 cps, about 1500 cps or about 1000 cps.

The pH of the viscoelastic composition of one embodiment is from a minimum of about 5 to a maximum of about 8. In one embodiment, the pH of the viscoelastic composition is from a minimum of about 5.5, about 6 or about 6.5 to a maximum of about 7.5, about 7.2 or about 7.

In a preferred embodiment, the viscoelastic composition comprises the following:
5.25 %w/v alginate
1%w/v vitamin E TPGS
Purified water q. s. a. d. to 100 %w/v

### Uses

Viscoelastic compositions according to any one or more of the foregoing embodiments, concepts or aspects including combinations and variations of the foregoing embodiments can be used according to the following method or methods.

In one embodiment, a method of maintaining space in a cavity in human tissue is described. The method comprises the step of injecting, into the cavity, a viscoelastic composition according to any embodiment, aspect, feature, combination or concept disclosed herein. Thereafter, the viscoelastic composition is removed from the cavity. Preferably, the cavity is the anterior chamber of the eye or the capsular bag.

In still another embodiment, a method of protecting tissue from trauma during a surgical procedure is disclosed. The method comprises the step of coating at least a portion of the tissue with a viscoelastic composition according to any embodiment, aspect, feature, combination or concept disclosed herein. Preferably, the tissue that is covered is in the anterior chamber of the eye and/or the capsular bag. A surgical procedure is then performed near the tissue. When the surgical procedure is completed, at least a portion of the viscoelastic composition is removed from the tissue.

in one embodiment, a method of replacing a natural lens from an eye is disclosed. Examples of procedures for removing a lens from a patient's eye include but are not limited to those disclosed in U.S. Patent Nos. 3,589,363 (cataract surgery), 3,693,613 (phacoemulsification) and 5,718,676 (process using micro flow needle), which are all incorporated herein by reference in their entirety. The process generally includes providing a passage through a sclera or cornea into an anterior chamber of the eye. The process involves making a small incision into the sclera or cornea. Alternatively or additionally, a cannula or trochar is used to create a passage through the sclera or cornea. Preferably, the incision or passage is as small as possible. Preferably, the incision or passage is smaller than about 5 mm, about 4 mm or about 3mm. Thereafter, the aqueous humor is withdrawn or otherwise removed from the anterior chamber of the eye.

A viscoelastic composition according to any one of the embodiments, aspects substantially concepts, combinations or features is inserted into the anterior chamber. The viscoelastic composition maintains the space in the anterior chamber. The viscoelastic composition coats the tissue in the wall of the anterior chamber.

According to one embodiment, a package for a viscoelastic composition that includes a delivery device is described. The device delivers a viscoelastic composition into the anterior chamber of a patient's eye. The device includes a syringe that contains a viscoelastic composition according to any embodiment, aspect, combination, concept or feature disclosed herein.

The syringe further comprises an outlet port and, optionally, a cannula configured to sealably connect to the outlet port. The cannula has a maximum inner diameter of about 2 mm. Typically, the maximum inner diameter is about 1.8 mm, about 1.5 mm or about 1 mm. Generally, the minimum inner diameter is about 0.8 mm, about 0.6 mm or about 0.4 mm.

In one embodiment, the viscoelastic composition requires a maximum force of 30 N to pass through a stainless steel cannula having a length of 2.2 cm and an inner diameter of 0.5 mm at a delivery rate of 0.02 ml/sec. Preferably, the viscoelastic composition requires a maximum force of about 27 N, about 25 N, about 20 N or about 18 N to pass through a stainless steel cannula having a length of 2.2 cm and an inner diameter of 0.5 mm at a delivery rate of 0.02 ml/sec.

Once the viscoelastic composition is inserted into the anterior chamber the crystalline lens is removed. The technique for removing the lens includes performing a capsulorhexis incision and breaking down the lens into smaller pieces through phacoemulsification or other known techniques. Thereafter, the pieces are removed by, for example, aspiration.

The viscoelastic composition is inserted into the capsular bag for space maintenance purposes. Moreover, the viscoelastic composition coats the capsular bag and protects it for additional steps in the surgical procedure.

According to one embodiment, the intraocular lens is inserted into the capsular bag. Typically, there is a method of inserting an intraocular lens into a capsular bag of an eye. The method comprises providing a lens insertion device comprising a loadable chamber configured to receive the intraocular lens, a tapered conduit having a first end connected to the loadable chamber and a second end. The second end is configured to penetrate through the passage in the corneal lens and into the capsular bag. An example of a lens insertion device is found in U.S. Patent No. 6,558,419, which is incorporated herein by reference in its entirety. The lens insertion device is further configured with a slidable actuator. The slidable actuator of one embodiment is configured to actuate the intraocular lens through the conduit past the second end. Typically, the second end of the tapered conduit has an inner diameter that is a maximum of about 5 mm. Preferably the second end of the tapered conduit has an inner diameter that is a maximum of about 4 mm substantially about 3.5 mm, about 3 mm or about 2.8 mm. Preferably, a maximum force of about 30 N is required to deliver the intraocular lens through the cannula. More preferably, a maximum force of about 27 N, about 25 N, about 20 N or about 18 N is required to deliver the intraocular lens through the cannula.

Prior to deployment, at least a portion of the intraocular lens is coated with a viscoelastic composition according to any one of the embodiments, aspects, concepts, combinations or features of the present invention. The intraocular lens is loaded into the loadable chamber either before or after it is coated. The conduit is inserted through the passage. The actuator forces the intraocular lens through the passage and into the capsular bag. After the intraocular lens is deployed, the conduit is removed from the passage.

Typically, at least a portion of the viscoelastic composition is removed from the capsular bag and/or anterior chamber. A physiological solution is then used to fill the anterior chamber. The sclera and/or cornea are sutured to close the passage.

Although preferred embodiments have been depicted and described in detail, it will be apparent to those skilled in the relevant art that the specification has been prepared without the intention of limiting the scope of the invention and that various modifications, additions, substitutions, and the like can be made without departing from the spirit of the invention and these are therefore considered to be within the scope of the invention as defined in the claims which follow.

## Claims

1. A viscoelastic composition comprising an aqueous solution having from a minimum of 0.01%w/v to a maximum of 20%w/v of an alginate viscoelastic polymer based upon the total volume of the viscoelastic composition and further having soluble vitamin derivative, wherein the soluble vitamin derivative includes Vitamin E TPGS or Vitamin E TPGSA.

2. The composition of claim 1, wherein the concentration of soluble vitamin derivative is from a minimum of 0.1 %w/v to a maximum of 4%w/v based upon the total weight of the viscoelastic composition.

3. The composition of claim 1, wherein the soluble vitamin derivative is present in the viscoelastic composition in a minimum amount of 0.2%w/v to a maximum amount of 3%w/v.

4. The composition of claim 1, wherein the ratio of the viscosity of the viscoelastic composition to the viscosity of a comparable viscoelastic composition having no soluble vitamin derivative is from a minimum of about 1 to a maximum of about 2.5.

5. The composition of claim 1, wherein the percentage of quenching is a minimum of about 45%.

6. The composition of claim 1, wherein the concentration of alginate viscoelastic polymer is from a minimum of 0.05%w/v to a maximum of 9%w/v based upon the volume of the viscoelastic composition.

7. The composition of claim 1, wherein the average molecular weight of the alginate composition is from a minimum of 50 kD to a maximum of 5,000 kD.

8. The composition of claim 1, wherein the osmolality of the viscoelastic composition is from a minimum of 200mOsmol/kg to a maximum of 400mOsmol/kg.

9. The composition of claim 1, wherein the zero-shear viscosity of the viscoelastic composition is from a minimum of about 6.10⁴ cps to a maximum of about 4.10⁶ cps.

10. The composition of claim 1, wherein the high-shear viscosity of the viscoelastic composition is from a minimum of about 500 cps to a maximum of about 2000 cps.

11. A viscoelastic composition as defined in any of claims 1 to 10 for use in a method of temporarily maintaining space in a cavity in human tissue, the method comprising the steps of:
(a) injecting the viscoelastic composition into the cavity; and
(b) removing the viscoelastic composition from the cavity.

12. The viscoelastic composition of claim 11, wherein the cavity is the anterior chamber of the eye or the capsular bag.

13. A viscoelastic composition as defined in any of claims 1 to 10 for use in a method of protecting tissue from trauma during a surgical procedure, the method comprising the steps of:
(a) coating at least a portion of the tissue with the viscoelastic composition;
(b) performing a surgical procedure near the tissue after the step of (a) coating; and
(c) removing at least a portion of the viscoelastic composition from the tissue after the step of (b) performing.

14. The viscoelastic composition of claim 13, wherein the step of (a) coating covers at least a portion of the tissue in an anterior chamber of an eye.

15. The viscoelastic composition of claim 13, wherein the step of (a) coating covers at least a portion of the tissue in a capsular bag of an eye.

16. A viscoelastic composition as defined in any of claims 1 to 10 for use in a method of replacing a natural lens from an eye, the method comprising the steps of:
(a) providing a passage through a sclera into an anterior chamber of the eye;
(b) removing at least a portion of the aqueous humor from the anterior chamber;
(c) inserting the viscoelastic composition into the anterior chamber;
(d) phacoemulsifying a lens in the capsular bag of the eye;
(e) removing substantially all of the lens from the capsular bag;
(f) injecting the viscoelastic composition into the capsular bag; and
(g) inserting an intraocular lens into the capsular bag.

17. The viscoelastic composition of claim 16, further comprising the step of (b) removing at least a portion of the viscoelastic composition from the capsular bag.

18. The viscoelastic composition of claim 16, further comprising the step of (b) removing at least a portion of the viscoelastic composition from the anterior chamber.

19. The viscoelastic composition claim 18, further comprising the step of suturing the sclera after the step of (g) inserting an intraocular lens.

20. The viscoelastic composition of claim 16, wherein the step of inserting comprises coating the intraocular lens with the viscoelastic composition and delivering the intraocular lens through a cannula.

21. The viscoelastic of composition of claim 20, wherein the cannula has a tip configured to be inserted into the capsular bag, wherein the tip of the cannula has an inner diameter that is a maximum of about 1 mm.

22. The viscoelastic composition claim 20, wherein the step of delivering the intraocular lens through the cannula requires a maximum force of about 30 N.

23. A package for a viscoelastic composition, the package comprising a syringe containing the viscoelastic composition of any of claim 1 to 10.

24. The package of claim 23, wherein the syringe has an outlet port, the package further comprises a cannula configured to sealably connect to the outlet port having a maximum inner diameter of about 2 mm.

25. The package of claim 23, wherein viscoelastic composition requires a maximum force of 30 N to pass through the cannula.

26. Use of alginate viscoelastic polymer for the preparation of a viscoelastic composition as defined in any of claims 1 to 10, wherein the viscoelastic composition is to be employed in a method of temporarily maintaining space in a cavity in human tissue, the method comprising the steps of:
(a) injecting the viscoelastic composition into the cavity; and
(b) removing the viscoelastic composition from the cavity.

27. Use of claim 26, wherein the cavity is the anterior chamber of the eye or the capsular bag.

28. Use of alginate viscoelastic polymer for the preparation of a viscoelastic composition as defined in any of claims 1 to 10, wherein the viscoelastic composition is to be employed in a method of protecting tissue from trauma during a surgical procedure, the method comprising the steps of:
(a) coating at least a portion of the tissue with the viscoelastic composition of claim 1;
(b) performing a surgical procedure near the tissue after the step of (a) coating; and
(c) removing at least a portion of the viscoelastic composition from the tissue after the step of (b) performing.

29. Use of claim 28, wherein the step of (a) coating covers at least a portion of the tissue in an anterior chamber of an eye.

30. Use of claim 28, wherein the step of (a) coating covers at least a portion of the tissue in a capsular bag of an eye.

31. Use of alginate viscoelastic polymer for the preparation of a viscoelastic composition as defined in any of claims 1 to 10, wherein the viscoelastic composition is to be employed in a method of replacing a natural lens from an eye, the method comprising the steps of:
(a) providing a passage through a sclera into an anterior chamber of the eye;
(b) removing at least a portion of the aqueous humor from the anterior chamber,
(c) inserting the viscoelastic composition of claim 1 into the anterior chamber;
(d) phacoemulsifying a lens in the capsular bag of the eye;
(e) removing substantially all of the lens from the capsular bag;
(f) injecting the viscoelastic composition into the capsular bag; and
(g) inserting an intraocular lens into the capsular bag.

32. Use of claim 31, further comprising the step of (b) removing at least a portion of the viscoelastic composition from the capsular bag.

33. Use of claim 31, further comprising the step of (b) removing at least a portion of the viscoelastic composition from the anterior chamber.

## Patentansprüche

1. Viskoelastische Zusammensetzung, umfassend eine wässrige Lösung mit mindestens 0,01% Gew./Vol. bis höchstens 20% Gew./Vol. eines viskoelastischen Alginatpolymers, bezogen auf das Gesamtvolumen der viskoelastischen Zusammensetzung, und weiter mit einem löslichen Vitamin-Derivat, wobei das lösliche Vitamin-Derivat Vitamin E TPGS oder Vitamin E TPGSA beinhaltet.

2. Zusammensetzung nach Anspruch 1, wobei die Konzentration an löslichem Vitamin-Derivat mindestens 0,1% Gew./Vol. bis höchstens 4% Gew./Vol., bezogen auf das Gesamtgewicht der viskoelastischen Zusammensetzung, beträgt.

3. Zusammensetzung nach Anspruch 1, wobei das lösliche Vitamin-Derivat in der viskoelastischen Zusammensetzung in einer Mindestmenge von 0,2% Gew./Vol. bis zu einer Höchstmenge von 3% Gew./Vol. vorliegt.

4. Zusammensetzung nach Anspruch 1, wobei das Verhältnis der Viskosität der viskoelastischen Zusammensetzung zur Viskosität einer vergleichbaren viskoelastischen Zusammensetzung ohne lösliches Vitamin-Derivat mindestens etwa 1 bis höchstens etwa 2,5 beträgt.

5. Zusammensetzung nach Anspruch 1, wobei der Prozentsatz des Quenchens mindestens etwa 45% beträgt.

6. Zusammensetzung nach Anspruch 1, wobei die Konzentration an viskoelastischem Alginatpolymer mindestens 0,05% Gew./Vol. bis höchstens 9% Gew./Vol., bezogen auf das Volumen der viskoelastischen Zusammensetzung, beträgt.

7. Zusammensetzung nach Anspruch 1, wobei das mittlere Molekulargewicht der Alginatzusammensetzung mindestens 50 kD bis höchstens 5000 kD beträgt.

8. Zusammensetzung nach Anspruch 1, wobei die Osmolalität der viskoelastischen Zusammensetzung mindestens 200 mOsmol/kg bis höchstens 400 mOsmol/kg beträgt.

9. Zusammensetzung nach Anspruch 1, wobei die Nullscherviskosität der viskoelastischen Zusammensetzung mindestens etwa 6 · 10⁴ cP bis höchstens etwa 4 · 10⁶ cP beträgt.

10. Zusammensetzung nach Anspruch 1, wobei die Hochscherviskosität der viskoelastischen Zusammensetzung mindestens etwa 500 cP bis höchstens etwa 2000 cP beträgt.

11. Viskoelastische Zusammensetzung nach einem der Ansprüche 1 bis 10 zur Verwendung in einem Verfahren zum vorübergehenden Platzhalten in einem Hohlraum in menschlichem Gewebe, wobei das Verfahren die Schritte umfasst:
(a) Einspritzen der viskoelastischen Zusammensetzung in den Hohlraum; und
(b) Entfernen der viskoelastischen Zusammensetzung aus dem Hohlraum.

12. Viskoelastische Zusammensetzung nach Anspruch 11, wobei der Hohlraum die vordere Augenkammer oder der Kapselsack ist.

13. Viskoelastische Zusammensetzung nach einem der Ansprüche 1 bis 10 zur Verwendung in einem Verfahren zum Schutz von Gewebe vor Trauma während eines chirurgischen Eingriffs, wobei das Verfahren die Schritte umfasst:
(a) Überziehen mindestens eines Teils des Gewebes mit der viskoelastischen Zusammensetzung;
(b) Durchführen eines chirurgischen Eingriffs in der Nähe des Gewebes nach dem Schritt des Überziehens (a); und
(c) Entfernen zumindest eines Teils der viskoelastischen Zusammensetzung von dem Gewebe nach dem Schritt des Durchführens (b).

14. Viskoelastische Zusammensetzung nach Anspruch 13, wobei der Schritt des Überziehens
(a) zumindest einen Teil des Gewebes in einer vorderen Augenkammer bedeckt.

15. Viskoelastische Zusammensetzung nach Anspruch 13, wobei der Schritt des Überziehens
(a) zumindest einen Teil des Gewebes in einem Kapselsack eines Auges bedeckt.

16. Viskoelastische Zusammensetzung nach einem der Ansprüche 1 bis 10 zur Verwendung in einem Verfahren zum Ersetzen einer natürlichen Linse eines Auges, wobei das Verfahren die Schritte umfasst:
(a) Bereitstellen eines Durchgangs durch eine Sklera in eine vordere Augenkammer;
(b) Entfernen zumindest eines Teils der wässrigen Flüssigkeit aus der vorderen Augenkammer;
(c) Einbringen der viskoelastischen Zusammensetzung in die vordere Augenkammer;
(d) Phakoemulgieren einer Linse im Kapselsack des Auges;
(e) Entfernen von im Wesentlichen der gesamten Linse aus dem Kapselsack;
(f) Einspritzen der viskoelastischen Zusammensetzung in den Kapselsack; und
(g) Einsetzen einer intraokulären Linse in den Kapselsack.

17. Viskoelastische Zusammensetzung nach Anspruch 16, weiter umfassend den Schritt (b) des Entfernens zumindest eines Teils der viskoelastischen Zusammensetzung aus dem Kapselsack.

18. Viskoelastische Zusammensetzung nach Anspruch 16, weiter umfassend den Schritt (b) des Entfernens zumindest eines Teils der viskoelastischen Zusammensetzung aus der vorderen Augenkammer.

19. Viskoelastische Zusammensetzung nach Anspruch 18, weiter umfassend den Schritt des Verschließens der Sklera nach dem Schritt (g) des Einsetzens einer intraokulären Linse.

20. Viskoelastische Zusammensetzung nach Anspruch 16, wobei der Schritt des Einbringens das Überziehen der intraokulären Linse mit der viskoelastischen Zusammensetzung und Zufuhr der intraokulären Linse durch eine Kanüle umfasst.

21. Viskoelastische Zusammensetzung nach Anspruch 20, wobei die Kanüle eine Spitze aufweist, die so geformt ist, dass sie in den Kapselsack eingebracht wird, wobei die Spitze der Kanüle einen Innendurchmesser von höchstens etwa 1 mm aufweist.

22. Viskoelastische Zusammensetzung nach Anspruch 20, wobei der Schritt der Zufuhr der intraokulären Linse durch die Kanüle eine maximale Kraft von etwa 30 N erfordert.

23. Packung für eine viskoelastische Zusammensetzung, wobei die Packung eine Spritze umfasst, die die viskoelastische Zusammensetzung nach einem der Ansprüche 1 bis 10 enthält.

24. Packung nach Anspruch 23, wobei die Spritze eine Auslassöffnung aufweist, die Packung weiter eine Kanüle umfasst, die so geformt ist, dass sie eine abdichtbare Verbindung mit der Auslassöffnung mit einem maximalen Innendurchmesser von etwa 2 mm eingeht.

25. Packung nach Anspruch 23, wobei die viskoelastische Zusammensetzung eine maximale Kraft von 30 N erfordert, um die Kanüle zu passieren.

26. Verwendung eines viskoelastischen Alginatpolymers zur Herstellung einer viskoelastischen Zusammensetzung nach einem der Ansprüche 1 bis 10, wobei die viskoelastische Zusammensetzung in einem Verfahren des vorübergehenden Platzhaltens in einem Hohlraum in menschlichem Gewebe eingesetzt werden soll, wobei das Verfahren die Schritte umfasst:
(a) Einspritzen der viskoelastischen Zusammensetzung in den Hohlraum; und
(b) Entfernen der viskoelastischen Zusammensetzung aus dem Hohlraum.

27. Verwendung nach Anspruch 26, wobei der Hohlraum die vordere Augenkammer oder der Kapselsack ist.

28. Verwendung eines viskoelastischen Alginatpolymers zur Herstellung einer viskoelastischen Zusammensetzung nach einem der Ansprüche 1 bis 10, wobei die viskoelastische Zusammensetzung in einem Verfahren zum Schutz von Gewebe vor Trauma während eines chirurgischen Eingriffs eingesetzt werden soll, wobei das Verfahren die Schritte umfasst:
(a) Überziehen zumindest eines Teils des Gewebes mit der viskoelastischen Zusammensetzung nach Anspruch 1;
(b) Durchführen eines chirurgischen Eingriffs in der Nähe des Gewebes nach dem Schritt des Überziehens (a); und
(c) Entfernen zumindest eines Teils der viskoelastischen Zusammensetzung von dem Gewebe nach dem Schritt des Durchführens (b).

29. Verwendung nach Anspruch 28, wobei der Schritt des Überziehens (a) zumindest einen Teil des Gewebes in einer vorderen Augenkammer bedeckt.

30. Verwendung nach Anspruch 28, wobei der Schritt des Überziehens (a) zumindest einen Teil des Gewebes in einem Kapselsack eines Auges bedeckt.

31. Verwendung eines viskoelastischen Alginatpolymers zur Herstellung einer viskoelastischen Zusammensetzung nach einem der Ansprüche 1 bis 10, wobei die viskoelastische Zusammensetzung in einem Verfahren zum Ersetzen einer natürlichen Linse eines Auges eingesetzt werden soll, wobei das Verfahren die Schritte umfasst:
(a) Bereitstellen eines Durchgangs durch eine Sklera in eine vordere Augenkammer;
(b) Entfernen zumindest eines Teils der wässrigen Flüssigkeit aus der vorderen Augenkammer;
(c) Einbringen der viskoelastischen Zusammensetzung nach Anspruch 1 in die vordere Augenkammer;
(d) Phakoemulgieren einer Linse im Kapselsack des Auges;
(e) Entfernen von im Wesentlichen der gesamten Linse aus dem Kapselsack;
(f) Einspritzen der viskoelastischen Zusammensetzung in den Kapselsack; und
(g) Einsetzen einer intraokulären Linse in den Kapselsack.

32. Verwendung nach Anspruch 31, weiter umfassend den Schritt (b) des Entfernens zumindest eines Teils der viskoelastischen Zusammensetzung aus dem Kapselsack.

33. Verwendung nach Anspruch 31, weiter umfassend den Schritt (b) des Entfernens zumindest eines Teils der viskoelastischen Zusammensetzung aus der vorderen Augenkammer.

## Revendications

1. Composition viscoélastique comprenant une solution aqueuse ayant un minimum de 0,01% p/v à un maximum de 20% p/v d'un alginate polymère viscoélastique basé sur le volume total de la composition viscoélastique et comprenant en outre un dérivé vitaminique soluble, dans laquelle le dérivé vitaminique soluble comprend la vitamine E TPGS ou la vitamine E TPGSA.

2. Composition selon la revendication 1, dans laquelle la concentration du dérivé vitaminique soluble va d'un minimum de 0,1% p/v à un maximum de 4% p/v basé sur le poids total de la composition viscoélastique.

3. Composition selon la revendication 1, dans laquelle le dérivé vitaminique soluble est présent dans la composition viscoélastique en une quantité minimale de 0,2% p/v à une quantité maximale de 3% p/v.

4. Composition selon la revendication 1, dans laquelle le rapport de la viscosité de la composition viscoélastique à la viscosité d'une composition viscoélastique comparable n'ayant aucun dérivé vitaminique soluble va d'un minimum d'environ 1 à un maximum d'environ 2,5.

5. Composition selon la revendication 1, dans laquelle le pourcentage d'affaiblissement est au minimum d'environ 45%.

6. Composition selon la revendication 1, dans laquelle la concentration de polymère viscoélastique d'alginate va d'un minimum de 0,05% p/v à un maximum de 9% p/v basé sur le volume de la composition viscoélastique.

7. Composition selon la revendication 1, dans laquelle la masse moléculaire moyenne de la composition d'alginate va d'un minimum de 50 kD à un maximum de 5 000 kD.

8. Composition selon la revendication 1, dans laquelle l'osmolalité de la composition viscoélastique va d'un minimum de 200 mOsmol/kg à un maximum de 400 mOsmol/kg.

9. Composition selon la revendication 1, dans laquelle la viscosité à un taux de cisaillement nul de la composition viscoélastique va d'un minimum d'environ 6.10⁴ cps à un maximum d'environ 4.10⁶ cps.

10. Composition selon la revendication 1, dans laquelle la viscosité à un taux de cisaillement élevé de la composition viscoélastique va d'un minimum d'environ 500 cps à un maximum d'environ 2 000 cps.

11. Composition viscoélastique telle que définie selon l'une quelconque des revendications 1 à 10 pour une utilisation dans un procédé de conservation temporaire d'espace dans une cavité à l'intérieur d'un tissu humain, le procédé comprenant les étapes consistant à :
(a) injecter la composition viscoélastique dans la cavité ; et
(b) retirer la composition viscoélastique de la cavité.

12. Composition viscoélastique selon la revendication 11, dans laquelle la cavité est la chambre antérieure de l'oeil ou le sac capsulaire.

13. Composition viscoélastique telle que définie selon l'une quelconque des revendications 1 à 10 pour une utilisation dans un procédé de protection d'un tissu envers une lésion pendant une intervention chirurgicale, le procédé comprenant les étapes consistant à :
(a) enrober au moins une partie du tissu avec la composition viscoélastique ;
(b) réaliser une intervention chirurgicale près du tissu après l'étape (a) de revêtement ; et
(c) retirer au moins une partie de la composition viscoélastique du tissu après l'étape (b) de réalisation.

14. Composition viscoélastique selon la revendication 13, dans laquelle l'étape (a) de revêtement recouvre au moins une partie du tissu dans une chambre antérieure d'un oeil.

15. Composition viscoélastique selon la revendication 13, dans laquelle l'étape (a) de revêtement recouvre au moins une partie du tissu dans un sac capsulaire d'un oeil.

16. Composition viscoélastique telle que définie selon l'une quelconque des revendications 1 à 10 pour une utilisation dans un procédé de remplacement d'un cristallin d'un oeil, le procédé comprenant les étapes consistant à :
(a) fournir un passage au travers d'une sclérotique dans une chambre antérieure de l'oeil ;
(b) retirer au moins une partie de l'humeur aqueuse de la chambre antérieure ;
(c) insérer la composition viscoélastique dans la chambre antérieure ;
(d) phacoémulsifier un cristallin dans le sac capsulaire de l'oeil ;
(e) retirer sensiblement tout le cristallin du sac capsulaire ;
(f) injecter la composition viscoélastique dans le sac capsulaire ; et
(g) insérer une lentille intra-oculaire dans le sac capsulaire.

17. Composition viscoélastique selon la revendication 16, comprenant en outre l'étape (b) d'élimination d'au moins une partie de la composition viscoélastique du sac capsulaire.

18. Composition viscoélastique selon la revendication 16, comprenant en outre l'étape (b) d'élimination d'au moins une partie de la composition viscoélastique de la chambre antérieure.

19. Composition viscoélastique selon la revendication 18, comprenant en outre l'étape de suture de la sclérotique après l'étape (g) d'insertion d'une lentille intra-oculaire.

20. Composition viscoélastique selon la revendication 16, dans laquelle l'étape d'insertion comprend l'enrobage de la lentille intra-oculaire avec la composition viscoélastique et l'acheminement de la lentille intra-oculaire au moyen d'une canule.

21. Composition viscoélastique selon la revendication 20, dans laquelle la canule présente une extrémité conçue pour être insérée dans le sac capsulaire où l'extrémité de la canule possède un diamètre interne d'un maximum d'environ 1 mm.

22. Composition viscoélastique selon la revendication 20, dans laquelle l'étape d'acheminement de la lentille intra-oculaire au moyen de la canule nécessite une force maximale d'environ 30 N.

23. Trousse pour une composition viscoélastique, la trousse comprenant une seringue contenant la composition viscoélastique selon l'une quelconque des revendications 1 à 10.

24. Trousse selon la revendication 23, dans laquelle la seringue possède un orifice de sortie, la trousse comprend en outre une canule conçue pour être connectée de manière étanche à l'orifice de sortie ayant un diamètre interne maximum d'environ 2 mm.

25. Trousse selon la revendication 23, dans laquelle la composition viscoélastique nécessite une force maximale de 30 N pour traverser la canule.

26. Utilisation d'un alginate polymère viscoélastique pour la préparation d'une composition viscoélastique telle que définie selon l'une quelconque des revendications 1 à 10, dans laquelle la composition
viscoélastique doit être employée dans un procédé de conservation temporaire d'espace dans une cavité à l'intérieur d'un tissu humain, le procédé comprenant les étapes consistant à :
(a) injecter la composition viscoélastique dans la cavité ; et
(b) retirer la composition viscoélastique de la cavité.

27. Utilisation selon la revendication 26, dans laquelle la cavité est la chambre antérieure de l'oeil ou le sac capsulaire.

28. Utilisation d'un alginate polymère viscoélastique pour la préparation d'une composition viscoélastique telle que définie selon l'une quelconque des revendications 1 à 10, dans laquelle la composition viscoélastique doit être employée dans un procédé de protection d'un tissu d'une lésion pendant une intervention chirurgicale, le procédé comprenant les étapes consistant à :
(a) revêtir au moins une partie du tissu avec la composition viscoélastique selon la revendication 1 ;
(b) réaliser une intervention chirurgicale près du tissu après l'étape (a) de revêtement ; et
(c) retirer au moins une partie de la composition viscoélastique du tissu après l'étape (b) de réalisation.

29. Utilisation selon la revendication 28, dans laquelle l'étape (a) de revêtement recouvre au moins une partie du tissu dans une chambre antérieure d'un oeil.

30. Utilisation selon la revendication 28, dans laquelle l'étape (a) de revêtement recouvre au moins une partie du tissu dans un sac capsulaire d'un oeil.

31. Utilisation d'un alginate polymère viscoélastique pour la préparation d'une composition viscoélastique telle que définie selon l'une quelconque des revendications 1 à 10, dans laquelle la composition viscoélastique doit être employée dans un procédé de remplacement d'un cristallin d'un oeil, le procédé comprenant les étapes consistant à :
(a) fournir un passage au travers d'une sclérotique dans une chambre antérieure de l'oeil ;
(b) retirer au moins une partie de l'humeur aqueuse de la chambre antérieure ;
(c) insérer la composition viscoélastique selon la revendication 1 dans la chambre antérieure ;
(d) phacoémulsifier un cristallin dans le sac capsulaire de l'oeil ;
(e) retirer sensiblement tout le cristallin du sac capsulaire ;
(f) injecter la composition viscoélastique dans le sac capsulaire ; et
(g) insérer une lentille intra-oculaire dans le sac capsulaire.

32. Utilisation selon la revendication 31, comprenant en outre l'étape (b) d'élimination d'au moins une partie de la composition viscoélastique du sac capsulaire.

33. Utilisation selon la revendication 31, comprenant en outre l'étape (b) d'élimination d'au moins une partie de la composition viscoélastique de la chambre antérieure.
